(19) Europäisches Patentamt
European Patent Office
Office européen des brevets

(11) Publication number: **0 000 663**
**B1**

(12) # EUROPEAN PATENT SPECIFICATION

(45) Date of publication of patent specification: **21.10.81**

(21) Application number: **78300199.3**

(22) Date of filing: **25.07.78**

(51) Int. Cl.³: **C 07 C 45/35,**
C 07 C 47/20,
C 07 C 47/22,
C 07 C 51/215,
C 07 C 57/04,
B 01 J 23/88, B 01 J 27/02,
B 01 J 27/18

(54) **Process for the oxidation of olefins using molybdenum containing catalysts containing various promoter elements.**

(30) Priority: **28.07.77 US 819733**

(43) Date of publication of application:
**07.02.79 Bulletin 79/3**

(45) Publication of the grant of the European patent:
**21.10.81 Bulletin 81/42**

(84) Designated Contracting States:
**BE CH DE FR GB NL**

(56) References cited:
FR - A - 2 138 601
FR - A - 2 227 257
FR - A - 2 228 538
FR - A - 2 232 524
FR - A - 2 302 993
FR - A - 2 303 781
FR - A - 2 308 609
FR - A - 2 333 770
FR - A - 2 354 812
NL - A - 75 08637
US - A - 4 001 317
US - A - 4 065 507

(73) Proprietor: **THE STANDARD OIL COMPANY**
**Midland Building**
**Cleveland, Ohio 44115 (US)**

(72) Inventor: **Guttmann, Andrew Tytus**
**5241 Philip Street**
**Warrensville Ohio (US)**
Inventor: **Grasselli, Robert Karl**
**150 Greentree Road**
**Chagrin Falls Ohio (US)**

(74) Representative: **Baillie, Iain Cameron et al,**
**c/o Ladas, Parry, von Gehr. Goldsmith &**
**Deschamps Blumenstrasse 48**
**D-8000 München 2 (DE)**

EP 0 000 663 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the oxidation of olefins using molybdenum containing catalysts containing various promoter elements

The process for oxidizing olefins by contacting the olefins together with an oxidizing agent with multicomponent catalysts is known. U.S. Patent No. 3,642,930 discloses that certain complex catalysts based on iron, bismuth and molybdenum can be employed in the oxidation of olefins to obtain unsaturated aldehydes and acids. Also, see U.S. Patent No. 4,001,317 which has a similar disclosure and British Patent No. 1,437,235, which discloses catalysts based on oxides of bismuth and molybdenum, which further contain at least one of indium, gallium, lanthanum and aluminum.

The catalysts described in these patents are indeed very desirable for the oxidation of olefins to unsaturated aldehydes and acids. Unfortunately, some of these catalysts exhibit a less than desired redox stability when subjected to stressful conditions. More specifically, it occasionally happens in a commercial facility that the amount of oxygen fed to the reactor along with the olefin feed is either much greater or much less than the desired value. When this happens, it has been found that the catalysts may exhibit a significant decrease in catalytic activity. This, of course, is very disadvantageous.

The present invention provides a new process for the catalytic oxidation of olefins to unsaturated aldehydes and acids which employs catalysts having high redox stability so that the catalysts can withstand major deviations in redox conditions without significant decrease in catalytic activity.

The process of the invention produces unsaturated aldehydes and acids by the vapor phase oxidation of propylene or isobutylene with molecular oxygen at a temperature of about 200° to 600°C. in the presence of a catalyst represented by the following formula:

$$A_aB_bFe_cX_dM_eMo_{12}O_x$$

wherein A is alkali metal, thallium, silver or mixtures thereof;

wherein B is cobalt, nickel, zinc, cadmium, beryllium, calcium, strontium, barium, radium or mixtures thereof;

X is Bi, Te or mixtures thereof; and

wherein M is selected from at least one of:

(1) a two-or-more-element system selected from Cr + W, Ge + W, Mn + Sb, Cr + P, Ge + P, Cu + W, Cu + Sn, Mn + Cr, Pr + W, Ce + W, Sn + Mn, Mn + Ge or combinations thereof;

(2) Pb, B, Cu or combinations thereof; and

(3) Mg + P, Mg + Cu, Mg + Cr, Mg + Cr + W, Mg + W, Mg + Sn, or combinations thereof; and further

wherein $0 \leqq a \leqq 5$, $0 \leqq b \leqq 20$, $0 \leqq c \leqq 20$, $0 \leqq d \leqq 20$, $0.01 \leqq e \leqq 12$, and provided that when M is Mn + Sb, *a* is greater than 0

x is a number such that the valence requirements for the other elements for oxygen are satisfied.

The one embodiment of the invention, the catalyst is free of indium, gallium, lanthanum and aluminium impurities when M is B, Cr + W, Pb, and/or Cu.

In another embodiment, the catalyst described is free of indium, gallium, lanthanum and aluminum impurities.

Preferably, the relative amounts of the various ingredients in the foregoing catalysts are such that the following inequalities apply: $0 \leqq a \leqq 0.5$, $0.1 \leqq b \leqq 20$, $0.1 \leqq c \leqq 20$, $0.1 \leqq d \leqq 20$ and $0.01 \leqq e \leqq 6$.

The catalysts of this invention preferably contain K, Rb and/or Cs. Also, in the catalysts of the invention X is preferably Bi.

In a particularly preferred embodiment, the catalysts employed in the inventive process are represented by the formula:

$$A_aB_bFe_cBi_cM_eMo_{12}O_x$$

wherein A is an alkali metal, preferably K, Rb, Cs or mixtures thereof,

B is Co, Ni or mixtures thereof; and

M is the same as described above; and further

$0.03 \leqq a \leqq 0.5$, $0.1 \leqq b \leqq 20$, $0.1 \leqq c \leqq 20$,

$0.1 \leqq d \leqq 20$, and $0.1 \leqq e \leqq 6$.

These catalysts are preferably free of In, Ga, La and Al impurities.

Of particular note are those catalysts falling within the foregoing generic descriptions in which M is selected from

Cr + W, Ge + W, Cr + P, Ge + P, Cu + W, Cu + Sn, Mn + Cr, Sn + Mn, Mn + Ge, Pb, B, Sn and Mg + Sn.

In the foregoing generic descriptions in which the M component is a specific two-or-three-element system as described in subparagraphs (1) and (3), the minimum amount of each element in the system is 1, preferably 5, atom percent based on the total number of atoms in the system.

Processes for the oxidation of propylene and/or isobutylene to form the corresponding unsaturated aldehydes and acids are well known in the art. Broadly, a mixture of the olefin and molecular oxygen, optionally in the presence of steam or other diluent, is contacted with a catalyst at an elevated temperature of about 200° to 600°C. for a contact time sufficient to convert the olefin to the desired aldehydes and/or acids. Normally, the products of these reactions contains a very large portion of the aldehyde and a small by-product amount of the unsaturated acid. The contact time may vary widely from a few seconds to ten or twenty seconds or more. The reaction can be conducted under atmospheric, superatmospheric or subatmospheic pressure with the use of a superatmospheric pressure normally being used on a commercial scale.

An important aspect of the present invention is the particular catalysts employed. The catalyst employed may be any of the catalysts delineated by the formula described above. Preferred are those catalysts falling within the foregoing generic description which contain potassium, rubidium, cesium or mixtures thereof and those contain cobalt or nickel or mixtures thereof, and catalysts containing potassium, rubidium, cesium or mixtures thereof as well as nickel or cobalt or mixtures thereof are particularly preferred.

The catalysts of the present invention can be prepared by techniques well known in the art. In this connection, techniques for preparing analogous catalysts are thoroughly described in the patents and application referred to abovce. Such catalysts are most conveniently prepared by the co-precipitation of soluble salts, although any other conventional technique can be employed. More specific information on the preparation of catalysts is given in the following examples.

The catalysts of the present invention may be employed in unsupported form or they may be supported on a suitable carrier. Suitable carriers include silica, alumina, Alundum, titania, zirconia, silicon carbide and the like. The catalysts may also be used in various physical forms. For example, the catalysts can be employed in a form suitable for carrying out the invention reaction in a fixed-bed mode or the catalyst can be employed in a form suitable for carrying out the invention reaction in a fluid-bed form.

As indicated above, a remarkable feature of the present invention is that the catalysts employed exhibit significant redox stability. In a commercial plant for producing unsaturated aldehydes and acids from propylene and isobutylene, mishaps inevitably occur. If the amount of molecular oxygen relative to the amount of olefin contacting the catalysts at any particular time significantly drops below the desired value, a noticeable decrease in catalytic activity of the catalyst may occur. In accordance with the present invention, the catalysts employed exhibit a far reduced tendency to lose their catalytic activity when subjected to unfavourable reaction conditions. From a commercial-standpoint, therefore, the inventive process using the catalysts described herein has significant advantages over presently commercially practiced processes.

## Examples

In order to more thoroughly illustrate the present invention, the following working examples are presented:

Various fixed-bed catalysts of the invention containing 20% $SiO_2$ were prepared by the procedures described below. Also prepared were a number of catalysts not included within the present invention, which were provided for comparative purposes.

*Reference catalyst A — 80% $K_{0.1}Ni_{2.5}Co_{4.5}Fe_3BiP_{0.5}Mo_{12}O_{12}O_x$ & 20% $SiO_2$*

An aqueous slurry (referred to a solution A) containing 37.00 grams $(NH_4)_6Mo_7O_{24}\cdot 4H_2O$, 8.56 grams of a 0.10 g./ml. aqueous solution of $H_3PO_4$, 38 ml. of water and 25.43 grams of a 40% silica sol was prepared. An aqueous solution (referred to as solution B) containing 21.17 grams $Fe(NO_3)_3\cdot 9H_2O$, 8.47 grams $Bi(NO_3)_3\cdot 5H_2O$, 12.7 grams $Ni(NO_3)_2\cdot 6H_2O$, 22.87 grams $Co(NO_3)_2\cdot 6H_2O$ and 1.75 ml. of a 0.10 g./ml. aqueous solution of $KNO_3$ was separately prepared. Solution A was then heated initially to 45—55°C. and solution B added dropwise to solution A with stirring. During addition of solution B, the temperature of the composition was increased so as to reach 75—80°C. at the end of the solution B addition. Stirring was continued and the temperature of the composition maintained between about 80 and 85°C. until sufficient water had evaporated so that a thick paste was obtained.

The thick paste was placed in an oven at 120°C. and heated for about $2\frac{1}{2}$ hours, the paste being stirred every $\frac{1}{2}$ hour. Heating was then continued until the paste was dry. The dried paste was then heated in air at 290°C. for 3 hours and then at 425°C. for 3 hours. The heated paste was then additionally heated in air at 550°C. for 16 hours to produce the indicated catalyst.

*Reference Catalyst B — 80% $K_{0.1}Ni_{2.5}Co_{4.5}Fe_3BiW_{0.5}Mo_{12}O_x$ & 20% $SiO_2$.*

The procedure described above for the preparation of Reference Catalyst A was repeated except that an appropriate amount of $(NH_4)_6W_7O_{24}\cdot 6H_2O$ was substituted for the $H_3PO_4$ in solution A.

*Catalysts 1 to 16*

Catalysts having the general formula:

$L_rK_{0.1}Ni_{2.5}Co_{4.5}Fe_qBiZ_{0.5}Mo_{12}$

wherein L is Cr, Ge, Mn or Cu;

Z is W, Sb, P, Sn, Cr, Pb, Ge or B; and

wherein q = 2 or 3;

r = 0 or 1; and

q + r = 3

were prepared by the general method described above in connection with the preparation of Reference Catalyst A. These catalysts, which are composed of a base catalyst $K_{0.1}Ni_{2.5}Co_{4.5}BiMo_{12}O_x$ and a promoter system $Fe_qL_rZ_{0.5}$, are described in the following Table I. In this table, only the promoters are identified, the catalysts of course being composed of the identified promoters plus the base catalyst.

*Oxidation of Propylene to Acrolein and Acrylic Acid*

In order to illustrate the excellent redox stability of the catalysts of the present invention when employed in the inventive process, each of the catalysts described in Table I was subjected to a redox test in the following manner. 5 cc. of each catalyst prepared above was charged into a fixed-bed reactor. The temperature of the catalyst in the reactor was raised to a predetermined value and a feed comprising propylene/oxygen (in the form of air)/water in a ratio of 1/2.3/4 was fed to the reactor at a rate such that the apparent contact time was 3 seconds and a WWH of about 0.07. Once the reaction had commenced, a sample of the product was recovered and analyzed for acrolein and acrylic acid so that the initial catalytic activity of the catalyst could be determined. Thereafter, the ratio of the ingredients in the feed as indicated above was changed to 1/0.7/4, and the temperature of the catalyst was raised to 400°C. This low oxygen was fed to the reactor under these conditions for a period of 2 hours. Next, the catalyst was reoxidized by feeding a feed of oxygen (in the form of air)/stem in a ratio of 2.3/4 to the catalyst at the reaction temperature indicated in Table I for 1 hour. Thereafter, the propylene flow was resumed to its initial value, and a product sample taken after the reaction had proceeded to steady state.

The results of these experiments are given in the following Table I. In this Table, the following definitions are used:

$$\% \text{ Per Pass Conversion} = \frac{\text{Moles of Propylene Reacted}}{\text{Moles of Propylene Fed}} \times 100$$

$$\% \text{ Selectivity} = \frac{\text{Moles of Product Formed}}{\text{Moles of Propylene Reacted}} \times 100$$

$$\text{Performance Number} = 1/2\ \{(\text{PPC to ACR.} + \text{AA}) + (\text{SEL. TO ACR.} + \text{AA})\}$$

In Table I, ACR is acrolein, and AA is acrylic acid. The performance number as defined above is a measure of the catalytic activity of a catalyst in that it is a function of both the selectivity and per pass conversion.

TABLE I

All Catalysts Supported on 20% $SiO_2$ (NALCO) Unless Stated Differently

| Example | Catalyst Promoter | Reaction Temp. °C. | Per Pass Conversion ACR. | AA | ACR+AA | Selectively ACR+AA | Performance No. Initial | Final | % Loss | % Improvement Over Reference Catalysts |
|---|---|---|---|---|---|---|---|---|---|---|
| (1) | REFERENCE CATALYSTS | | | | | | | | | |
| | A $Fe_3P_{0.5}$ | 350 | 80.0 | 9.5 | 89.5 | 91.4 | 90 | 66 | –26.7 | — |
| | A $Fe_3P_{0.5}$ | 350 | 79.0 | 9.4 | 88.4 | 90.7 | 90 | 72 | –20.0 | — |
| | B $Fe_3W_{0.5}$ | 350 | 80.3 | 8.9 | 89.2 | 92.0 | 91 | 75 | –19.0 | + 8.7 |
| (2) | IMPROVED REDOX CATALYSTS | | | | | | | | | |
| | (A) Double Substituted | | | | | | | | | |
| | 1 $Fe_2CrW_{0.5}$ | 350 | 80.4 | 7.7 | 88.1 | 92.9 | 91 | 89 | –2.2 | +29.0 |
| | 2 $Fe_2CrW_{0.5}$ | 380 | 75.1 | 15.4 | 90.5 | 93.1 | 92 | 90 | –2.2 | +30.4 |
| | 3 $Fe_2GeW_{0.5}$ | 380 | 74.0 | 12.1 | 86.1 | 93.5 | 90 | 89 | –1.1 | +29.0 |
| | 4 $Fe_2MnSb_{0.5}$ | 350 | 76.3 | 12.0 | 88.3 | 92.1 | 90 | 86 | –4.4 | +24.6 |
| | 5 $Fe_2CrP_{0.5}$ | 380 | 76.0 | 10.8 | 86.8 | 90.0 | 88 | 84 | –4.5 | +21.7 |
| | 6 $Fe_2GeP_{0.5}$ | 380 | 79.6 | 8.9 | 88.5 | 93.0 | 91 | 86 | –5.5 | +24.6 |
| | 7 $Fe_2CuW_{0.5}$ | 320 | 75.2 | 2.9 | 78.1 | 96.9 | 87 | 81 | –6.9 | +17.4 |
| | 8 $Fe_2CuW_{0.5}$ | 350 | 73.4 | 6.8 | 80.2 | 93.9 | 87 | 80 | –8.0 | +15.9 |
| | 9 $Fe_2CuSn_{0.5}$ | 350 | 78.7 | 6.9 | 85.6 | 93.3 | 89 | 80 | –10.1 | +15.9 |
| | 10 $Fe_2MnCr_{0.5}$ | 320 | 79.4 | 4.6 | 85.5 | 93.1 | 89 | 80 | –10.1 | +15.9 |
| | 11 $Fe_2MnCr_{0.5}$ | 350 | 80.3 | 11.1 | 91.4 | 92.5 | 92 | 81 | –12.0 | +17.4 |
| | 12 $Fe_2CrW_{0.5}$ | 320 | 83.2 | 6.4 | 89.6 | 92.8 | 91 | 81 | –11.0 | +17.4 |

TABLE I (cont.)

All Catalysts Supported on 20% $Sio_2$ (NALCO) Unless Stated Differently

| Example | Catalyst Promoter | Reaction Temp. °C. | Per Pass Conversion ACR. | AA | ACR+AA | Selectivity ACR+AA | Performance No. Initial | Final | % Loss | % Improvement Over Reference Catalysts |
|---|---|---|---|---|---|---|---|---|---|---|
| | (B) Single Substituted | | | | | | | | | |
| 13 | $Fe_3Pb_{0.5}$ | 320 | 76.6 | 4.3 | 80.9 | 89.1 | 85 | 74 | −12.9 | + 7.3 |
| 14 | $Fe_3B_{0.5}$ | 350 | 79.1 | 11.8 | 90.9 | 90.9 | 91 | 79 | −13.2 | +14.5 |
| | (C) Mg-Containing Systems | | | | | | | | | |
| 15 | $Mg_{7.5}Fe_3R_{0.5}$ | 380 | 71.0 | 6.9 | 77.9 | 91.1 | 84 | 86 | + 2.4 | +24.6 |
| 16 | $Mg_{7.5}Fe_{3.5}W_{0.5}$ | 380 | 64.5 | 4.4 | 68.9 | 95.6 | 82 | 83 | + 1.2 | +20.3 |

From the foregoing; it can be seen that the catalysts of the present invention in the inventive reaction show a much smaller loss in performance number (and indeed some of the catalysts even show an improvement in performance number) over the reference catalysts. This means that the inventive catalysts when employed in the inventive reaction exhibit a far greater redox stability when subjected to unfavorable reaction conditions as compared to conventional catalysts.

Although only a few embodiments of the present invention have been described above, it should be appreciated that many modifications can be made without departing from the spirit and scope of the invention.

## Claims

1. A process for the preparation of unsaturated aldehydes and acids from propylene or isobutylene by the vapor phase oxidation of propylene or isobutylene with molecular oxygen at a temperature of about 200° to 600°C. in the presence of a molybdenum-containing catalyst characterized in that the molybdenum-containing catalyst is one of the formula

$$A_aB_bFe_cX_dM_eMo_{12}O_x$$

wherein A is alkali metal, thallium, silver or mixtures thereof:

wherein B is cobalt, nickel, zinc, cadmium, beryllium, calcium, strontium, barium, radium or mixtures thereof;

X is bismuth, tellurium or mixtures thereof; and

wherein M is one or more of the following:

(1) a two-or-more-element system selected from Cr + W, Ge + W, Mn + Sb, Cr + P, Ge + P, Cu + W, Cu + Sn, Mn + Cr, Pr + W, Ce + W, Sn + Mn, Mn + Ge or combinations thereof;

(2) Pb, B, Cu or combinations thereof; and

(3) a two-or-more-element system selected from Mg + P, Mg + Cu, Mg + Cr, Mg + Cr + W, Mg + W, Mg + Sn or combinations thereof; and further

wherein $0 \leqq a \leqq 5$, $0 \leqq b \leqq 20$, $0 \leqq c \leqq 20$, $0 \leqq d \leqq 20$, $0.01 \leqq e \leqq 12$, and provided that when M is Mn + Sb, *a* is greater than 0

x is a number such that the valence requirements for the other elements for oxygen are satisfied, and wherein the minimum amount of each element in M when M is a combination of two or more elements in one atom percent based on the number of atoms in component M.

2. A process according to claim 1, characterized in that the catalyst is free of In, Ga, La and Al impurities when M is B, Cr + W, Pb, Cu or mixtures thereof.

3. A process according to claim 2, characterized in that M is selected from Cr + W, Ge + W, Cr + P, Ge + P, Cu + W, Cu + Sn, Mn + Cr, Sn + Mn, Mn + Ge, Pb, B, and Mg + Sn.

4. A process according to any one of claims 1 to 3, characterized in that X is Bi.

5. A process according to any one of the preceding claims, characterized in that the elements of said catalyst are present such that $0 \leqq a \leqq 0.5$, $0.1 \leqq b \leqq 20$, $0.1 \leqq c \leqq 20$, $0.1 \leqq d \leqq 20$ and $0.01 \leqq e \leqq 6$, provided that when M is Mn + Sb, *a* is greater than 0.

6. A process according to any one of the preceding claims, characterized in that A is at least one of K, Rb, and Cs and further wherein B is Ni + Co.

7. A process according to any one of claims 1, 2, 4, 5 or 6, characterized in that M is Mn + Sb.

8. A process according to any one of claims 1, 2, 4, 5 or 6, characterized in that M is Ge + W.

9. A process according to claims 1 or 3, characterized in that A is alkali metal, B is Co, Ni or mixtures thereof, X is Bi

$$0.03 \leqq a \leqq 5$$
$$0.1 \leqq b \leqq 20$$
$$0.1 \leqq c \leqq 20$$
$$0.1 \leqq d \leqq 20, \text{ and}$$
$$0.1 \leqq e \leqq 6.$$

10. A process according to claim 9, characterized in that said catalyst is free of In, Ga, La and Al impurities.

11. A process according to claim 1, characterized in that M is one or more of the following two-or-more-element systems selected from

(1) Pr + W, Ce + W,

(2) Mg + P, Mg + Cu, Mg + Cr, Mg + Cr + W, Mg + W, or combinations thereof.

12. A process according to claim 1, characterized in that M is Mg + W.

13. A process according to claim 1, characterized in that M is Ge + P.

14. A process according to claim 1, characterized in that M is Cr + W.

15. A process according to claim 1, characterized in that M is Cr + P.

## Revendications

1. Procédé de préparation d'aldéhydes et d'acides insaturés à partir de propylène ou d'isobutylène, par oxydation en phase vapeur de propylène ou d'isobutylène avec de l'oxygène moléculaire à une température d'environ 200° à 600°C, en présence d'un catalyseur contenant du molybdène, caractérisé en ce que le catalyseur contenant du molybdène est un de formule

$$A_aB_bFe_cX_dM_eMo_{12}O_x$$

où A est un métal alcalin, du thallium, de l'argent ou un de leurs mélanges;

B est le cobalt, le nickel, le zinc, le cadmium, le béryllium, le calcium, le strontium, le baryum, le radium ou leurs mélanges;

X est le bismuth, le tellure ou une de leurs mélanges; et M est un ou plusieurs des systèmes suivants:

(1) système à deux ou plusieurs éléments choisis entre Cr + W, Ge + W, Mn + Sb, Cr + P, Ge + P, Cu + W, Cu + Sn, Mn + Cr, Pr + W, Ce + W, Sn + Mn, Mn + Ge ou leurs combinaisons;

(2) Pb, B, Cu ou leurs combinaisons; et

(3) un système à deux ou plusieurs éléments choisi entre Mg + P, Mg + Cu, Mg + Cr, Mg + Cr + W, Mg + W, Mg + Sn ou leurs combinaisons; et en outre

où $0 \leqq a \leqq 5$, $0 \leqq b \leqq 20$, $0 \leqq c \leqq 20$, $0 \leqq d \leqq 20$, $0{,}01 \leqq e \leqq 12$,

et pourvu, que, quand M est Mn + Sb, *a* soit supérieur à 0,

x est un nombre tel que les exigences de valence pour les autres éléments vis-à-vis de l'oxygène soient satisfaites, et où la quantité minimale de chaque élément dans M, quand M est une combinaison de deux ou plusieurs éléments, est un atome pour cent par rapport au nombre d'atomes dans le composant M.

2. Procédé selon la revendication 1, caractérisé en ce que le catalyseur est exempt d'impuretés indium, gallium, lanthane et aluminium quand M est B, Cr + W, Pb, Cu ou leurs mélanges.

3. Procédé selon la revendication 2, caractérisé en ce que M est choisi entre Cr + W, Ge + W, Cr + P, Ge + P, Cu + W, Cu + Sn, Mn + Cr, Sn + Mn, Mn + Ge, Pb, B, et Mg + Sn.

4. Procédé selon l'une quelconque des revendications 1, 2 et 3, caractérisé en ce que X est Bi.

5. Procédé selon l'une quelconque des revendications 1 à 4, caractérisé en ce que les éléments dudit catalyseur sont présents de telle sorte que $0 \leqq a \leqq 0{,}5$, $0{,}1 \leqq b \leqq 20$, $0{,}1 \leqq c \leqq 20$, $0{,}1 \leqq d \leqq 20$ et $0{,}01 \leqq e \leqq 6$, pourvu que quand M est Mn + Sb, a soit supérieur à 0.

6. Procédé selon l'une quelconque des revendications 1 à 5, caractérisé en ce que A est au moins l'un de K, Rb et Cs et en outre en ce que B est Ni + Co.

7. Procédé selon l'une quelconque des revendications 1, 2, 4, 5 et 6, caractérisé en ce que M est Mn + Sb.

8. Procédé selon l'une quelconque des revendications 1, 2, 4, 5 et 6, caractérisé en ce que M est Ge + W.

9. Procédé selon l'une ou l'autre des revendications 1 et 3, caractérisé en ce que A est un métal alcalin, B est Co, Ni ou un de leurs mélanges, X est Bi,

$$0{,}03 \leqq a \leqq 5$$
$$0{,}1 \leqq b \leqq 20$$
$$0{,}1 \leqq c \leqq 20$$
$$0{,}1 \leqq d \leqq 20, \text{ et}$$
$$0{,}1 \leqq e \leqq 6.$$

10. Procédé selon la revendication 9, caractérisé en ce que ledit catalyseur est exempt d'impuretés indium, gallium, lanthane et aluminium.

11. procédé selon la revendication 1, caractérisé en ce que M est un ou plusieurs des systèmes à deux ou plusieurs éléments suivants, choisis entre

(1) Pr + W, Ce + W,

(2) Mg + P, Mg + Cu, Mg + Cr, Mg + Cr + W, Mg + W, ou leurs combinaisons.

12. Procédé selon la revendication 1, caractérisé en ce que M est Mg + W.

13. Procédé selon la revendication 1, caractérisé en ce que M est Ge + P.

14. Procédé selon la revendication 1, caractérisé en ce que M est Cr + W.

15. Procédé selon la revendication 1, caractérisé en ce que M est Cr + P.

## Patentansprüche

1. Verfahren zur Herstellung von ungesättigten Aldehyden und Säuren aus Propylen oder Isobutylen durch Oxidation von Propylen oder Isobutylen in der Dampfphase mit molekularem Sauerstoff bei einer Temperatur von etwa 200 bis 600°C in Anwesenheit eines molybdänhaltigen Katalysators, dadurch gekennzeichnet, daß der molybdänhaltige Katalysator die Formel

$$A_aB_bFe_cX_dN_eMo_{12}O_x$$

hat, wobei

A Alkalimetall, Thallium, Silber oder ein Gemisch derselben ist,
B Kobalt, Nickel, Zink, Cadmium, Beryllium, Calcium, Strontium, Barium, Radium oder ein Gemisch derselben ist,
X Wismut, Tellur oder ein Gemisch derselben ist und
M mindestens eins der folgenden Komponenten ist:
(1) ein aus zwei oder mehr Elementen bestehendes System ausgewählt aus Cr + W, Ge + W, Mn + Sb, Cr + P, Ge + P, Cu + W, Cu + Sn, Mn + Cr, Pr + W, Ce + W, Sn + Mn, Mn + Ge oder Kombinationen derselben,
(2) Pb, B, Cu oder Kombibationen derselben und
(3) ein aus zwei oder mehr Elementen bestehendes System ausgewählt aus Mg + P, Mg + Cu, Mg + Cr, Mg + Cr + W, Mg + W, Mg + Sn oder Kombinationen derselben, und $0 \leqq a \leqq 5$, $0 \leqq b \leqq 20$, $0 \leqq c \leqq 20$, $0 \leqq d \leqq 20$, $0{,}01 \leqq 2 \leqq 12$ und wenn M Mn + Sb ist, a größer ist als 0.,
x eine solche Zahl ist, daß die Wertigkeitsanforderungen der anderen Elemente für Sauerstoff befriedigt sind, und die Mindestmenge jedes Elements von M, wenn M eine Kombination von zwei oder mehr elementen ist ein Atomprozent bezogen auf die Anzahl der Atome der Komponente M beträgt.

2. Verfahren nach Anspruch 1, dadurch gekennzeichnet, daß der Katalyst frei ist von den Verunreinigungen In, Ga, La und Al, wenn M B, Cr + W, Pb, Cu oder ein Gemisch derselben ist.

3. Verfahren nach Anspruch 2, dadurch gekennzeichnet, daß M ausgewählt ist aus Cr + W, Ge + W, Cr + P, Ge + P, Cu + W, Cu + Sn, Mn + Cr, Sn + Mn, Mn + Ge, Pb, B und Mg + Sn.

4. Verfahren nach einem der Ansprüche 1 + 3, dadurch gekennzeichnet, daß x Bi ist.

5. Verfahren nach einem der vorhergehenden Ansprüche, dadurch gekennziechnet, daß die Elemente des genannten Katalysators in solchen Anteilen vorhanden sind, daß $0 \leqq a \leqq 0{,}5$, $0{,}1 \leqq b \leqq 20$, $0{,}1 \leqq c \leqq 20$, $0{,}1 \leqq d \leqq 20$ und $0{,}01 \leqq e \leqq 6$, und daß, wenn M Mn + Sb ist, a größer ist als 0.

6. Verfahren nach einem der vorhergehenden Ansprüche, dadurch gekennzeichnet, daß A mindestens eines der Elemente K, Rb und Cs ist und daß B Ni + Co ist.

7. Verfahren nach einem der Ansprüche 1, 2, 4, 5 oder 6, dadurch gekennzeichnet, daß M Mn + Sb ist.

8. Verfahren nach einem der Ansprüche 1, 2, 4, 5 oder 6, dadurch gekennzeichnet, daß M Ge + W ist.

9. Verfahren nach Anspruch 1 oder 3, dadurch gekennzeichent, daß A Alkalimetall ist, B Co, Ni oder ein Gemisch derselben ist, X Bi ist,

$$0{,}03 \leqq a \leqq 5$$
$$0{,}1 \leqq b \leqq 20$$
$$0{,}1 \leqq c \leqq 20$$
$$0{,}1 \leqq d \leqq 20 \text{ und}$$
$$0{,}1 \leqq a \leqq 6 \text{ ist.}$$

10. Verfahren nach Anspruch 9, dadurch gekennzeichnet, daß der genannte Katalysator frei ist von den Verunreinigungen In, Ga, La und Al.

11. Verfahren nach Anspruch 1, dadurch gekennzeichnet, daß M aus mindestens einem der folgenden aus mindestens zwei Elementen bestehenden Systeme ist, die ausgewählt sind aus
(1) Pr + W, Ce + W
(2) Mg + P, Mg + Cu, Mg + Cr, Mg + Cr + W, Mg + W oder Kombinationen derselben.

12. Verfahren nach Anspruch 1, dadurch gekennzeichnet, daß M Mg + W ist.

13. Verfahren nach Anspruch 1, dadurch gekennzeichnet, daß M Ge + P ist.

14. Verfahren nach Anspruch 1, dadurch gekennzeichnet, daß M Cr + W ist.

15. Verfahren nach Anspruch 1, dadurch gekennzeichnet, daß M Cr + P ist.